# EUROPEAN PATENT SPECIFICATION

(11) **EP 1 240 120 B1**
(45) Date of publication and mention of the grant of the patent: **08.06.2005**
(21) Application number: 00985554.5
(22) Date of filing: 14.12.2000
(51) Int. Cl.: C07C 5/48

(54) **PROCESS FOR THE PRODUCTION OF OLEFINS**
VERFAHREN ZUR HERSTELLUNG VON OLEFINEN
PROCEDE DE PRODUCTION D'OLEFINES

(30) Priority: 23.12.1999 GB 9930598; 16.03.2000 GB 0006386
(43) Date of publication of application: 18.09.2002
(73) Proprietor: BP Chemicals Limited, London EC2M 7BA (GB)
(72) Inventor: COUVES, John William, High Wycombe, Buckinghamshire HP10 0QN (GB); GRIFFITHS, David Charles, Esher, Surrey KT10 0HU (GB); MESSENGER, Brian Edward, Englefield Green, Surrey TW20 OJQ (GB); REID, Ian, Allan Beattie, London SW18 5RX (GB)
(74) Representative: Hymers, Ronald Robson
(86) International application number: PCT/GB2000/004794
(87) International publication number: WO 2001/047842

(56) References cited:
- WO-A-00/14037
- WO-A-94/04632
- US-A- 3 437 703
- US-A- 4 788 371

## Description

The present invention relates to a process for the production of olefins.

Olefins such as ethylene and propylene may be produced by a variety of processes, including the steam cracking of hydrocarbons or by the dehydrogenation of paraffinic feedstocks. More recently, olefins have been produced by a process known as auto-thermal cracking. In such a process, a hydrocarbon feed is mixed with an oxygen-containing gas and contacted with a catalyst. The hydrocarbon feed is partially combusted, and the heat produced is used to drive the dehydrogenation reaction.

An example of an auto-thermal cracking process is described in EP 0 332 289. The patent describes platinum group metals as being capable of supporting combustion beyond the fuel rich limit of flammability. Preferred catalysts are supported platinum catalysts such as platinum/gamma alumina spheres, and platinum/monoliths such as platinum/cordierite or mullite monoliths.

In WO 97/26987, such platinum catalysts are modified with Sn or Cu.

In WO 00/14035, platinum catalysts are modified with tin, copper or antimony. Specifically, Pt/Sn, Pt/Cu, Pt/Sb and Pt/Sn/Sb catalysts are disclosed.

We have now developed other catalysts for auto-thermal cracking.

Accordingly, the present invention provides a process for the production of an olefin from a hydrocarbon, which process comprises:
partially combusting the hydrocarbon and an oxygen-containing gas in the presence of a catalyst, characterised in that the catalyst comprises platinum and at least one further metal, said further metal being selected from Ga, In, Ge and Bi. For the avoidance of doubt, the platinum and at least one further metal may be present in the catalyst in any form, for example, in metallic form, or in the form of a metal compound, such as an oxide.

The partial combustion reaction is carried out by contacting a feed comprising the hydrocarbon and a molecular oxygen containing gas with the catalyst. Any suitable oxygen-containing gas may be employed; oxygen being preferred.

The preferred stoichiometric ratio of hydrocarbon to oxygen is 5 to 16, preferably, 5 to 13.5 times, more preferably, 6 to 10 times the stoichiometric ratio of hydrocarbon to oxygen required for complete combustion of the hydrocarbon to carbon dioxide and water.

Preferably, hydrogen is co-fed into the reaction. It is believed that in the presence of the catalyst, hydrogen combusts preferentially relative to the hydrocarbon, thereby increasing the olefin selectivity of the overall process.

Additional feed components such as nitrogen, carbon monoxide and steam may also be fed into the reaction.

In one embodiment of the present invention, the catalyst comprises only one metal selected from Ga, In, Ge and Bi.

In such an embodiment, platinum may nominally form between 0.01 and 5.0 wt %, preferably, between 0.01 and 2.0 wt %, and more preferably, between 0.05 and 1.0 wt % of the total weight of the catalyst. It should be noted, however, that not all the metal employed during the preparation of the catalyst necessarily becomes incorporated in the catalyst composition. Thus, the actual loading of metal may differ from the nominal loading. To ensure that the desired actual metal concentrations are achieved, the nominal metal concentrations may have to be varied accordingly.

The actual loading of platinum may be between 10 and up to 100% of the nominal loading. Preferably, the actual loadings are above 40%, more preferably, above 70 % (eg 90 to 99%) of the nominal values. In a preferred embodiment, platinum may actually form between 0.01 and 5.0 wt %, preferably, between 0.01 and 2.0 wt %, and more preferably, between 0.05 and 1.0 wt % of the total weight of the catalyst. The atomic ratio of platinum to the Ga, In, Ge or Bi may be 1 : 0.1 - 50.

Preferred atomic ratios of Pt:Ga are 1:0.1 - 20.0, more preferably, 1:0.2 - 15.0, even more preferably, 1:1.0 - 8.0, most preferably, 1:5.0 - 8.0, for example, 1:7.5. Preferred atomic ratios of Pt:In are 1:0.1 - 10.0, more preferably, 1: 0.2 - 7.0, and most preferably, 1:0.8 - 3.0., for example, 1:1.3.

Preferred atomic ratios of Pt:Ge are 1:0.1 - 20.0, more preferably, 1:0.2 - 15.0 and most preferably, 1:0.5 - 12, for example, 1:10.8.

Preferred atomic ratios of Pt:Bi are 1: 0.1 - 20.0, more preferably, 1:0.2 - 15.0 and most preferably, 1:0.5 - 10.0.

In another embodiment, the catalyst comprises at least two metals selected from Group IIIA, Group IVA, VA and the transition metal series. For example, the catalyst may comprise Pt, Pd and Sn or Pt, Pd and Cu. Platinum may nominally form between 0.01 and 5 wt %, preferably, between 0.01 and 2.0 wt %, and more preferably, 0.05 - 1.0 wt % of the total weight of the catalyst. As described above, the actual loading of metal may differ from the nominal loading. Thus, the actual loading of platinum may be between 10 and up to 100% of the nominal value. Preferably, the actual loadings are above 40%, more preferably, above 70 % (eg 90 - 99%) of the nominal values. In a preferred embodiment, the actual loading of platinum is between 0.01 and 5 wt %, preferably, between 0.01 and 2.0 wt %, and more preferably, 0.05 - 1.0 wt % of the total weight of the catalyst.

Preferably, the catalyst comprises a) platinum, b) a further transition metal and c) a Group IIIA or IVA metal. The transition metal b) is preferably palladium. The metal c) is, preferably, a Group IVA metal, more preferably Sn or Ge. The atomic ratio of metal b) (eg palladium) to platinum may be 1: 0.1 - 10.0, preferably, 1:0.5 - 8.0, and more preferably, 1: 1.0 -5.0. The atomic ratio of metal b) (eg palladium) to metal c) (eg Sn or Cu) may be 1: 0.1 - 60, preferably, 1: 0.1 - 50, for example, 1: 0.1 - 12. Where metal b) is Pd and metal c) is Sn, the Pd:Sn atomic ratio is preferably 1 : 0.1 - 60, more preferably, 1: 0.1 - 50, for example, 1 : 0.1 -12.0. Where metal b) is Pd, and metal c) Cu, the atomic Pd:Cu ratio is preferably 1: 0.1 - 3.0, more preferably, 1: 0.2 - 2:0, and most preferably, 1: 0.5 - 1.5.

The catalyst employed may be unsupported. For example, the catalyst may be in the form of a metal gauze. Preferably, however, the catalyst employed in the present process is a supported catalyst. The catalyst may be supported on any suitable support. Ceramic supports are generally preferred, although metal supports may also be employed.

Where ceramic supports are used, the composition of the ceramic support may be any oxide or combination of oxides that is stable at high temperatures of, for example, 600°C and 1200°C. The support material preferably has a low thermal expansion coefficient, and is resistant to phase separation at high temperatures.

Suitable ceramic supports include lithium aluminium silicate (LAS), alumina (α-Al₂O₃), yttria stabilised zirconia, alumina titanate, niascon, and calcium zirconyl phosphate. The supports may be wash-coated, for example, with γ-Al₂O₃.

The structure of the support material is important, as this may affect flow patterns through the catalyst. Such flow patterns may influence the transport ofreactants and products to and from the catalyst surface, thereby affecting the catalyst's activity. Preferably, the substrate is a continuous multi-channel ceramic structure, such as a foam, a regular channelled monolith or a fibrous pad. The pores of foam monolith structures tend to provide tortuous paths for reactants and products. Such supports may have 20 to 80, preferably, 30 to 50 pores per inch. Channel monoliths generally have straighter, channel-like pores. These pores are generally smaller, and there may be 80 or more pores per linear inch of catalyst. The support may be in the form of spheres or other granular shapes, or may be present as a thin layer or wash coat on another substrate.

The catalyst employed in the present invention may comprise further elements, such as alkali metals. Suitable alkali metals include lithium, sodium, potassium and caesium.

The catalyst employed in the present invention may be prepared by any method known in the art. For example, gel methods and wet-impregnation techniques may be employed. Typically, the support is impregnated with one or more solutions comprising the metals, dried and then calcined in air. The support may be impregnated in one or more steps. Preferably, multiple impregnation steps are employed. The support is preferably dried and calcined between each impregnation, and then subjected to a final calcination, preferably, in air. The calcined support may then be reduced, for example, by heat treatment in a hydrogen atmosphere.

In the case of a catalyst comprising platinum, palladium and tin, the support material is preferably impregnated in a platinum/palladium solution, and then in a solution comprising tin. Once impregnated, the catalyst is dried at eg 50 to 200°C, and calcined at eg 100 to 700°C e.g. 300 to 700°C,between each impregnation. The support is then subjected to a final calcination step at eg 400 to 1500°C in air. The catalyst may then be reduced in, for example, a hydrogen atmosphere. This reduction step may be carried out at temperatures of up to 1000°C, for example, 300 to 750 °C are employed.

The catalyst may be in the form of a fluidised or fixed bed. Preferably, a fixed bed catalyst is employed.

The partial combustion reaction may be suitably carried out at a catalyst exit temperature of between 600°C and 1200°C, preferably between 850°C and 1050°C and most preferably, between 900°C and 1000°C.

The reaction may be carried out at atmospheric or elevated pressure. Suitable pressures range from 0 to 2 bara, preferably 1.5 to 2 bara, for example 1.8 bara. Elevated pressures of for example, 2 to 50 bara, may also be suitable.

The hydrocarbon may comprise any suitable hydrocarbon. Preferably, gaseous hydrocarbons are employed. Suitable gaseous hydrocarbons include ethane, propane, butane and mixtures thereof.

The hydrocarbon is passed over the catalyst at a gas hourly space velocity of greater than 10,000 h⁻¹, preferably above 20,000 h⁻¹ and most preferably, greater than 100,000 h⁻¹. It will be understood, however, that the optimum gas hourly space time velocity will depend upon the pressure and nature of the feed composition.

Advantageously, heat may also be supplied by pre-heating the hydrocarbon. The temperature to which the hydrocarbon, oxygen-containing gas and (optionally) hydrogen mixture may be preheated, however, is limited by the autoignition properties (eg temperature) of the feed.

Where the cracking reaction is carried out at elevated pressure, the reaction products may be quenched as they emerge from the reaction chamber to avoid further reactions taking place.

Any coke produced in the process of the present invention may be removed by mechanical means, or using one of the decoking methods described in EP 0 709 446.

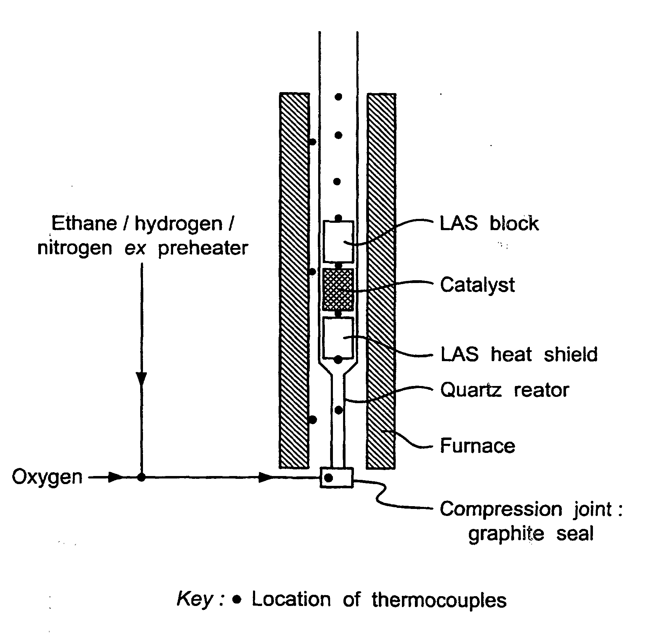

These and other aspects of the present invention will now be described, by way of example, with reference to Figure 1 of the drawings, which is a schematic view of an apparatus suitable for carrying out an embodiment of the present invention.

Figure 1 depicts an apparatus 10 comprising a reactor 12 surrounded by a furnace 14. The reactor 12 may be formed of quartz or metal. Where a metal reactor 12 is employed, the inside of the reactor is lined with quartz (not shown). Typically, metal reactors are more susceptible to heat loss than quartz reactors.

The reactor 12 is coupled to an oxygen supply 16 and a hydrocarbon feed supply 18. The hydrocarbon feed comprises ethane, and hydrogen and nitrogen. A catalyst 20 is located within the reactor 12. The catalyst 20 is positioned between a pair of LAS heat shields 22, 24.

The furnace is set to minimise heat losses, and the reactants 16, 18 are introduced into the reactor via line 26. As the reactants contact the catalyst 20, some of the ethane in the hydrocarbon feed 18 combusts to produce water and carbon oxides. The hydrogen co-feed also combusts to produce water. Both these combustion reactions are exothermic, and the heat produced is used to drive the dehydrogenation of ethane to ethylene.

### Examples

### Example 1 - Preparation of Pd/Pt/Sn Catalyst

The catalyst was prepared by multiple impregnation of a lithium aluminium silicate support having a high purity alumina (HPA) wash-coat. The support was impregnated in 1) a platinum/palladium solution ((NH₃)₄Pt^{II}Cl₂, (NH₃)₄Pd^{II}Cl₂), and 2) an SnCl₂/HCl solution. Between each impregnation, the support was dried at 120°C, and calcined at 450°C . The catalyst was then calcined in air at 600°C for 6 hours, and then reduced in an atmosphere of hydrogen (1.0nl/min), and nitrogen (1.5 nl/min) for 1 hour (at 700°C).

The catalyst was analysed and found to have 0.36 wt % Pt, 0.04 wt % Pd and 1.85 wt % Sn.

### Comparative Example A - 1 wt% Pt

A catalyst having a nominal loading of 1 wt % Pt was prepared by impregnating a lithium aluminium silicate support having an HPA wash-coat in a solution of (NH₃)₄Pt^{II}Cl₂. The impregnated support was dried at 120°C, and calcined at 450°C . The catalyst was then calcined in air at 1200°C for 6 hours. The catalyst was analysed and found to have 0.86 wt % Pt.

### Comparative Example B - Pt/Sn

A catalyst comprising Pt and Sn was prepared by impregnating a lithium aluminium silicate support having an HPA wash-coat in a solution of 1) (NH₃)₄Pt^{II}Cl₂, and 2) SnCl₂/HCl. The impregnated support was dried, calcined and reduced as described in connection with Example 1 above. The catalyst was analysed and found to have 0.48 wt % Pt, and 2.80 wt % Sn.

### Example 2

The catalysts of Example 1, Comparative Example A and Comparative Example B above were each tested as catalysts for the oxidative dehydrogenation of ethane. Each catalyst was mounted in the apparatus of Figure 1, and an oxidative dehydrogenation reaction was carried out under the conditions summarised in Table 1 below. For the tests, a metal reactor 12 was employed.

**Table 1**

| | 1 (Pd/Pt/Sn) | A (Pt only) | B (Pt/Sn) |
|---|---|---|---|
| GHSV @ stp/h | 120863 | 119759 | 120268 |
| ethane flow (g/min) | 18.07 | 17.28 | 18.07 |
| hydrogen flow (g/min) | 1.18 | 1.21 | 1.18 |
| oxygen flow (g/min) | 9.39 | 9.68 | 9.39 |
| nitrogen flow (g/min) | 4.95 | 4.52 | 4.72 |

As shown in Table 2 below, the selectivity of the catalyst of Example 1 towards ethylene is greater than those of Comparative Examples A and B, respectively.

**Table 2**

| | 1 (Pd/Pt/Sn) | A (Pt only) | B (Pt/Sn) |
|---|---|---|---|
| ethane conversion (%) | 74.70 | 75.69 | 73.03 |
| selectivity (g ethylene per 100g ethane converted) | 73.93 | 67.54 | 69.7 |

### Example 3 - Preparation of Pt/Ge catalyst

The catalyst was prepared by sequential impregnation of 30ppi ceramic foam blocks (lithium aluminium silicate with alumina washcoat) with aqueous tetrammineplatinum(II) chloride and germanium tetrachloride in ethanol. Between impregnations the blocks were dried in air at 120-140°C for ca. 30 minutes then calcined in air at 450°C for 30 minutes, cooled to room temperature and the impregnation procedure was repeated. Once all the impregnation solution had been absorbed onto the foam the blocks were calcined in air at 600°C for 6 hours.

Several of the calcined blocks were given a further air calcination at 1200°C for 6 hours before testing. Subsequent to air calcination at 600-1200°C the blocks could be reduced in a hydrogen (1.0 nl/min) and nitrogen (1.5 nl/min) atmosphere at 750°C for 1 hour prior to testing. The catalyst comprised 1.0 wt % Pt and 4.0 wt % Ge.

### Example 4

The catalyst of Example 3 was tested as a catalyst for the oxidative dehydrogenation of ethane using the apparatus of Figure 1. The reactor employed was formed of quartz. The performance of this catalyst was compared to that of Comparative Example A. The dehydrogenation conditions employed are summarised in Table 3 below. Table 4 shows the ethane conversion and selectivities achieved.

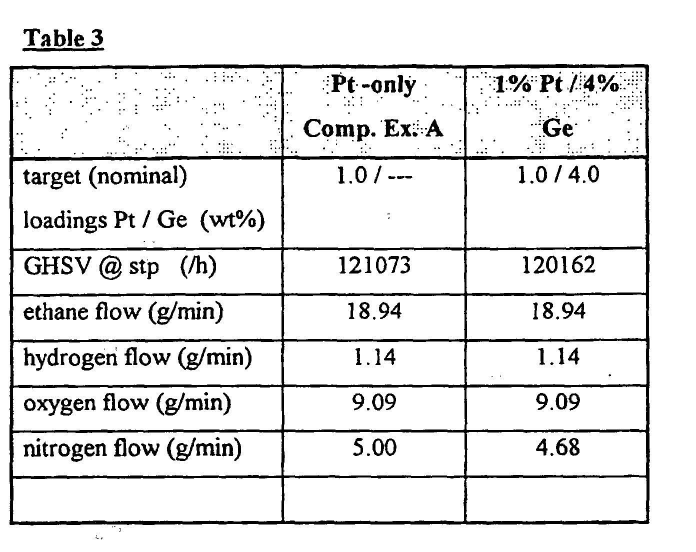

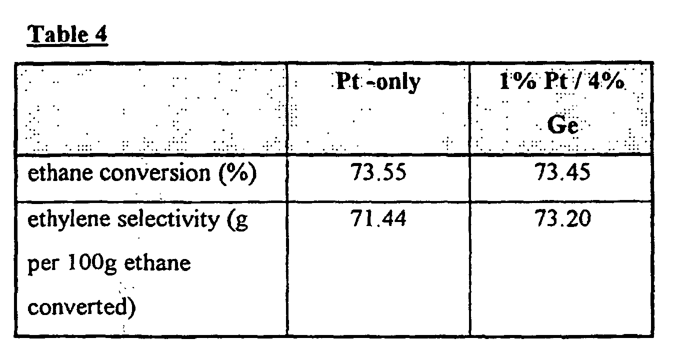

### Example 5 - Preparation of a Pt/Ga catalyst

The catalyst was prepared by sequential impregnation of 30ppi ceramic foam block (99.5% alumina with alumina washcoat) with aqueous tetrammineplatinum(II) chloride and aqueous gallium nitrate. The foam block employed was 28mm diameter and 30mm in depth. Between impregnations, the block was dried in air at 120-140°C for ca. 30 minutes, calcined in air at 450°C for 30 minutes, and then cooled to room temperature. Once all the impregnation solution had been absorbed onto the foam block, the block was calcined in air at 600°C for 6 hours.

The Pt and Ga solutions were prepared with sufficient salt to achieve final Pt/Ga loadings of 0.28 wt% platinum and 0.75 wt% gallium. The atomic platinum:gallium ratio was 1:7.5.

Prior to testing the catalysts were given an *in situ* reduction at 750°C under flowing hydrogen (ca. 1.0 nl/min) and nitrogen (ca. 1.5 nl/min) for 1 hour.

### Example 6

The catalysts of Example 5, and Comparative Example A were tested as catalysts for the oxidative dehydrogenation of ethane. Each catalyst was mounted in the apparatus of Figure 1, and an oxidative dehydrogenation reaction was carried out under the conditions summarised in Table 5 below.

**Table 5**

| | 5 (Pt/Ga) | A (Pt only) |
|---|---|---|
| GHSV @ stp/h | 121172 | 120130 |
| ethane flow (g/min) | 14.15 | 13.50 |
| hydrogen flow (g/min) | 12.73 | 13.17 |
| oxygen flow (g/min) | 6.37 | 6.58 |
| nitrogen flow (g/min) | 4.06 | 3.74 |

As shown in Table 6 below, the selectivity of the catalyst of Example 5 towards ethylene is greater than that of Comparative Example A.

**Table 6**

| | 5 (Pt/Ga) | A (Pt only) |
|---|---|---|
| ethane conversion (%) | 77.71 | 79.64 |
| selectivity (g ethylene per 100g | 71.85 | 68.96 |
| ethane converted) | | |

### Example 7 - Preparation of a Pt/In catalyst

The catalyst was prepared by sequential impregnation of 30ppi ceramic foam blocks (99.5% alumina with alumina washcoat) with aqueous tetrammineplatinum(II) chloride and aqueous indium nitrate. The foam block used was 28mm in diameter and 30mm in depth. Between impregnations, the block was dried in air at 120-140°C for ca. 30 minutes, calcined in air at 450°C for 30 minutes, and then cooled to room temperature. Once all the impregnation solution had been absorbed onto the foam the blocks were calcined in air at 600°C for 6 hours.

The Pt and In solutions were prepared with sufficient salt to achieve final Pt/In loadings of 0.54 wt% platinum and 0.4 wt% indium. The catalyst had an atomic platinum:indium ratio of 1:1.26

Prior to testing the catalysts were given an *in situ* reduction at 750°C under flowing hydrogen (ca. 1.0 nl/min) and nitrogen (ca. 1.5 nl/min) for 1 hour .

### Example 8

In this Example, a Pt/In catalyst was prepared in the manner of Example 7 above, except that the catalyst was calcined in air at 1200°C for 6 hours, rather than at 600°C.

### Example 9

The catalysts of Example 7, 8 and Comparative Example A were tested as catalysts for the oxidative dehydrogenation of ethane. Each catalyst was mounted in the apparatus of Figure 1, and an oxidative dehydrogenation reaction was carried out under the conditions summarised in Table 7 below.

**Table 7**

| | 7 (Pt/In) | 8 (Pt/In) | A (Pt only) |
|---|---|---|---|
| GHSV @ stp/h | 119975 | 119908 | 120130 |
| ethane flow (g/min) | 13.50 | 13.50 | 13.50 |
| hydrogen flow (g/min) | 13.17 | 13.17 | 13.17 |
| oxygen flow (g/min) | 6.58 | 6.58 | 6.58 |
| nitrogen flow (g/min) | 3.69 | 3.67 | 3.74 |

As shown in Table 8 below, the catalysts of Examples 7 and 8 show higher selectivities towards ethylene than the catalyst of Comparative Example A.

**Table 8**

| | 7 (Pt/In) | 8 (Pt/In) | A (Pt only) |
|---|---|---|---|
| ethane conversion (%) | 78.86 | 77.57 | 79.64 |
| selectivity (g ethylene per 100g ethane converted) | 70.19 | 71.27 | 68.96 |

## Claims

1. A process for the production of an olefin from a hydrocarbon, which process comprises:
partially combusting the hydrocarbon and an oxygen-containing gas in the presence of a catalyst, **characterised in that** the catalyst comprises platinum and at least one further metal, said further metal being selected from Ga, In, Ge and Bi.

2. A process as claimed in claim 1, wherein stoichiometric ratio of hydrocarbon to oxygen is 5 to 13.5 times, preferably, 6 to 10 times the stoichiometric ratio of hydrocarbon to oxygen required for complete combustion of the hydrocarbon to carbon dioxide and water.

3. A process as claimed in claim 1, wherein said partial combustion of said hydrocarbon and oxygen-containing gas is carried out in the presence of hydrogen.

4. A process as claimed in claim 1, wherein said further metal is either Ga or In.

5. A process as claimed in claim 1, wherein said further metal is Bi.

6. A process as claimed in claim 1 wherein said catalyst is a supported catalyst.

7. A process as claimed in claim 1 wherein said hydrocarbon comprises at least one hydrocarbon selected from the group consisting of ethane, propane and butane.

## Patentansprüche

1. Verfahren zur Herstellung eines Olefins aus einem Kohlenwasserstoff, wobei das Verfahren umfasst:
teilweises Verbrennen des Kohlenwasserstoffs und eines Sauerstoff-enthaltenden Gases in Gegenwart eines Katalysators, **dadurch gekennzeichnet, dass** der Katalysator Platin und mindestens ein weiteres Metall umfasst, wobei das weitere Metall aus Ga, In, Ge und Bi ausgewählt ist.

2. Verfahren nach Anspruch 1, wobei das stöchiometrische Verhältnis von Kohlenwasserstoff zu Sauerstoff das 5- bis 13,5fache, vorzugsweise 6- bis 10-fache des stöchiometrischen Verhältnisses von Kohlenwasserstoff zu Sauerstoff ist, das für eine vollständige Verbrennung des Kohlenwasserstoffs zu Kohlendioxid und Wasser erforderlich ist.

3. Verfahren nach Anspruch 1, wobei die teilweise Verbrennung des Kohlenwasserstoffs und Sauerstoff-enthaltenden Gases in Gegenwart von Wasserstoff ausgeführt wird.

4. Verfahren nach Anspruch 1, wobei das weitere Metall entweder Ga oder In ist.

5. Verfahren nach Anspruch 1, wobei das weitere Metall Bi ist.

6. Verfahren nach Anspruch 1, wobei der Katalysator ein getragener Katalysator ist.

7. Verfahren nach Anspruch 1, wobei der Kohlenwasserstoff mindestens einen Kohlenwasserstoff, ausgewählt aus der Gruppe, bestehend aus Ethan, Propan und Butan, umfasst.

## Revendications

1. Procédé pour la production d'une oléfine à partir d'un hydrocarbure, procédé qui comprend :
la combustion partielle de l'hydrocarbure et d'un gaz contenant de l'oxygène en présence d'un catalyseur, **caractérisé en ce que** le catalyseur comprend du platine et au moins un métal supplémentaire, ledit métal supplémentaire étant choisi entre Ga, In, Ge et Bi.

2. Procédé suivant la revendication 1, dans lequel le rapport stoechiométrique de l'hydrocarbure à l'oxygène est de 5 à 13,5 fois, de préférence 6 à 10 fois le rapport stoechiométrique de l'hydrocarbure à l'oxygène requis pour la combustion totale de l'hydrocarbure en dioxyde de carbone et eau.

3. Procédé suivant la revendication 1, dans lequel ladite combustion partielle dudit hydrocarbure et dudit gaz contenant de l'oxygène est effectuée en présence d'hydrogène.

4. Procédé suivant la revendication 1, dans lequel ledit métal supplémentaire est Ga ou In.

5. Procédé suivant la revendication 1, dans lequel ledit métal supplémentaire est Bi.

6. Procédé suivant la revendication 1, dans lequel ledit catalyseur est un catalyseur fixé sur un support.

7. Procédé suivant la revendication 1, dans lequel ledit hydrocarbure comprend au moins un hydrocarbure choisi dans le groupe consistant en éthane, propane et butane.
